# EUROPEAN PATENT SPECIFICATION

(11) **EP 1 537 842 B1**
(45) Date of publication and mention of the grant of the patent: **12.06.2013**
(21) Application number: 03791214.4
(22) Date of filing: 13.08.2003
(51) Int. Cl.: A61F 13/49, A61F 13/496

(54) **DISPOSABLE WEARING ARTICLE**
TRAGBARER EINWEGARTIKEL
ARTICLE VESTIMENTAIRE JETABLE

(30) Priority: 30.08.2002 JP 2002252970
(43) Date of publication of application: 08.06.2005
(73) Proprietor: UNI-CHARM CO., LTD., Kawanoe, Ehime 799-0111 (JP)
(72) Inventor: OTSUBO, Toshifumi, Uni-Charm Co., Ltd., Mitoyo-gun, Kagawa 769-1602 (JP); OHIRO, Masaya, Uni-Charm Co., Ltd., Mitoyo-gun, Kagawa 769-1602 (JP)
(74) Representative: Sperling, Rüdiger
(86) International application number: PCT/JP2003/010314
(87) International publication number: WO 2004/019844

(56) References cited:
- EP-A1- 1 016 395
- EP-A2- 0 865 780
- EP-A2- 0 933 073
- JP-A- 6 030 963
- JP-A- 10 258 082
- JP-A- 60 194 947

## Description

### TECHNICAL FIELD OF THE INVENTION

The present invention relates to a disposable wearing article for absorption and containment of bodily discharges.

### RELATED ART

Japanese Patent Application Publication No. 1998-258082A discloses a disposable diaper comprising a liquid-pervious topsheet, a liquid-impervious backsheet and a liquid-absorbent core interposed between these top- and backsheets. This diaper has first end portions extending outward beyond longitudinally opposite ends of the core lying in front and rear waist regions and second end portions extending further outward beyond the first end portions in a longitudinal direction. Each of the first end portions is provided with a waist-surrounding elastic member extending in a waist-circumferential direction so as to be contractible in the waist-circumferential direction, each of the second end portions is folded back onto inner surface of associated one of the first end portions and distal ends of the second end portions are secured to the first end portions so as to cover the waist-surrounding member.

The first and second end portions are defined by portions of the top- and backsheets extending outward beyond the longitudinally opposite ends of the core. The first and second portions are joined together only at the associated distal ends of the second end portions and free from each other in other zones. The first end portion is formed substantially over its entire area with a plurality of gathers as the waist-surrounding elastic member contracts inward as viewed in the waist-circumferential direction. In the front and rear waist regions, lateral marginal portions of the top- and backsheets extending outward beyond the side edges of the core in the waist-circumferential direction are overlaid and joined together by means of a plurality of heat-sealing lines arranged intermittently in the longitudinal direction. This diaper has a pants-like shape having a waist-hole and a pair of leg-holes. This diaper has advantageous effects that an uncomfortable rough touch against a wearer and stimulate a wearer' s skin can be reduced since the gathers (fine wrinkles) formed along the first end portions are covered with the second end portions.

In spite of such advantageous effects, the diaper disclosed in the above-cited Publication is still accompanied with problems. Specifically, the first end portions formed with a plurality of gathers come in direct contact with the wearer' s skin as the diaper is put on the wearer's body because the second end portions are folded back onto the outer surfaces of the first end portions. The gathers formed along the first end portions come in contact with the wearer's skin as the first end portions come in direct contact with the wearer's skin. Consequently, these gathers may cause to create an uncomfortable feeling against the wearer and sometimes leave compression marks on the wearer's skin. In addition, a gap may be left between the gathers formed along the first end portions and the wearer's skin through which an excessive amount of bodily discharges which can not be absorbed by the core may leak out from the diaper.

It is a first object of the present invention to provide a disposable wearing article improved so that any deterioration of the article's touch due to gathers can be avoided and none of compression marks due to the gathers is left on the wearer's skin even if the end portions of the front and rear waist regions are formed with a plurality of gathers as the waist-surrounding elastic member contracts. It is a second object of the present invention to provide a disposable wearing article improved so that the end portions of the front and rear waist regions can be held in sufficiently close contact with the wearer's skin to prevent bodily discharges from leaking beyond the end portions of the front and rear waist regions.

### DISCLOSURE OF THE INVENTION

According to the present invention, there is provided a the disposable wearing article generally comprising a base sheet assembly defining front and rear waist regions and a crotch region extending between the waist regions and a liquid-absorbent panel attached to an inner side of the base sheet assembly so as to extend over the crotch region further into the front and rear waist regions, the base sheet assembly having first end portions extending outward beyond longitudinally opposite ends of the panel lying in the front and rear waist regions in a longitudinal direction and second end portions extending further outward beyond the first end portions in the longitudinal direction, each of the first end portions being provided with a waist-surrounding elastic member extending in a waist-circumferential direction in a stretched state, each of the second end portions being folded back onto an inner surface of associated one of the first end portions and distal end zones of the second end portions extending in the waist-circumferential direction being secured to the first end portions so that the second end portions cover the waist-surrounding member.

The present invention further comprises the first and second end portions respective having lateral zones lying outside transversely opposite side edges of the panel as viewed in the waist-circumferential direction and intermediate zones bordered by the distal end zones and the lateral zones and the first and second end portions are bonded together in the lateral zones and let free from each other in the intermediate zones.

The present invention includes the following embodiments.

The first and second end portions are partially joined in the respective intermediate zones.

The base sheet assembly comprises a first sheet facing the panel and a second sheet facing away from the panel and the second portions are formed by at least one of the first and second sheets.

The waist-surrounding elastic member is interposed between the first and second sheets.

The first and second sheets constituting the front and rear waist regions of the base sheet assembly are joined together in the vicinity of peripheral edges thereof lying outside the transversely opposite lateral side edges of the panel and the wearing article is formed with a waist-hole and a pair of leg-holes.

The panel comprises a liquid-pervious topsheet facing the wearer's body, a liquid-impervious backsheet facing away from the wearer's body and a liquid-absorbent core interposed between the top- and backsheets.

### BRIEF DESCRIPTION OF THE DRAWINGS

Fig. 1 is a perspective view showing an example of the wearing article;
Fig. 2 is a partially cutaway developed plan view showing the article of Fig. 1 with front and rear waist regions disconnected from each other;
Fig. 3 is a sectional view taken along a line A-A in Fig. 2;
Fig. 4 is a sectional view taken along a line B-B in Fig. 2;
Fig. 5 is a sectional view taken along a line C-C in Fig. 1;
Fig. 6 is a developed plan view showing another example of the wearing article;
Fig. 7 is a sectional view taken along a line D-D in Fig. 6;
Fig. 8 is a sectional view taken along a line E-E in Fig. 6; and
Fig. 9 is a sectional view taken along a line F-F in Fig. 6.

### DESCRIPTION OF THE BEST MODE FOR WORKING OF THE INVENTION

Detail of the pants-type disposable wearing article according to the present invention will be more fully understood from the description given hereunder with reference to the accompanying drawings.

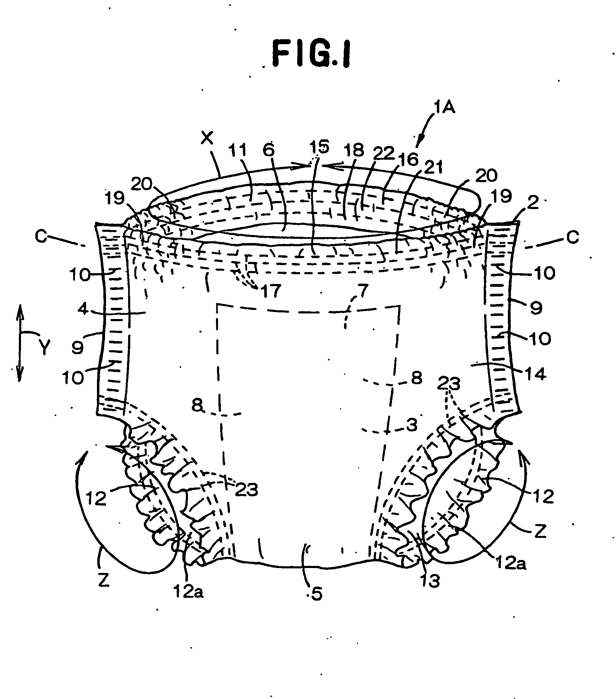

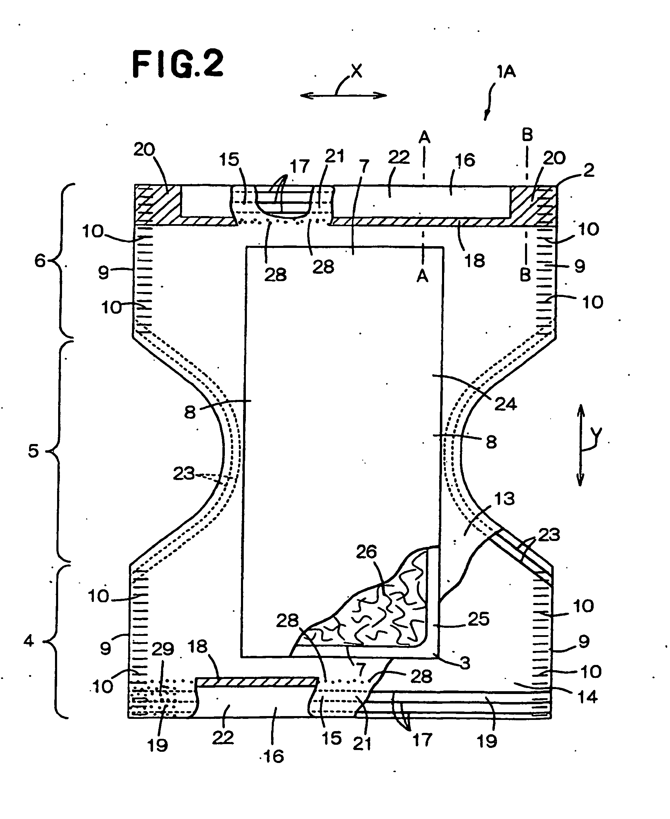

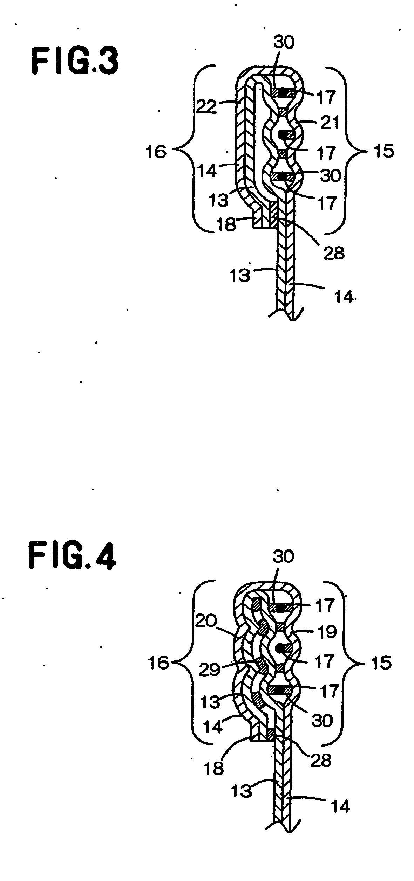

Fig. 1 is a perspective view showing a pants-type wearing article 1A according to a typical embodiment of the invention, Fig. 2 is a partially cutaway developed plan view showing the article 1A of Fig. 1 with front and rear waist regions 4, 6 disconnected from each other, Fig. 3 is a sectional view taken along a line A-A in Fig. 2 and Fig. 4 is a sectional view taken along a line B-B in Fig. 2. In Figs. 1 and 2, a waist-circumferential direction is indicated by an arrow X, a longitudinal direction is indicated by an arrow Y and a leg-circumferential direction is indicated by an arrow Z (in Fig. 1 alone). Expression "inner surfaces of inner and outer sheets 13, 14 (corresponding to first and second sheets) and upper and lower sheets 24, 25" should be understood to mean the surfaces of these sheets 13, 14 facing a core 26 and expression "outer surfaces of these sheets 13, 14, 24, 25" should be understood to mean the surfaces of these sheets 13, 14, 24, 25 facing away from the core 26.

The article 1A comprises a base sheet assembly 2 and a liquid-absorbent panel 3 attached to an inner side of the base sheet assembly 2. The base sheet assembly 2 defines front and rear waist regions 4, 6 opposed to each other and a crotch region 5 extending between these waist regions 4, 6. The panel 3 extends between the front and rear waist regions 4, 6 of the base sheet assembly 2 and have longitudinally opposite ends 7 lying in the front and rear waist regions 4, 6 of the base sheet assembly 2, respectively, so as to extend in the waist-circumferential direction and transversely opposite side edges 8 extending between the front and rear waist regions 4, 6 of the base sheet assembly 2 in the longitudinal direction.

Individual sheets constituting the base sheet assembly 2 are overlaid and joined together in the vicinity of lateral marginal edges 9 by means of a plurality of heat-sealing lines 10 arranged intermittently in the longitudinal direction. The article 1A is in the form of pants having a waist-hole 11 and a pair of leg-holes 12. The crotch region 5 describes circular arcs which are convex inward in the waist-circumferential direction of the article 1A. The base sheet assembly 2 presents a substantially hourglass-like planar shape as will be seen in Fig. 2.

The base sheet assembly 2 comprises the inner sheet 13 (first sheet) facing the panel 3 and the outer sheet 14 (second sheet) facing away from the panel 3, i.e., lying outside the inner sheet 13. The inner sheet 13 and the outer sheet 14 are made of hydrophobic fibrous nonwoven fabric. The inner sheet 13 and the outer sheet 14 are joined by means of hot melt adhesive (not shown) intermittently applied on at least one of these sheets 13, 14.

The base sheet assembly 2 has first end portions 15 extending outward beyond longitudinally opposite ends 7 of the panel 3 in the longitudinal direction and second end portions 16 extending further outward beyond the respective first end portions 15 in the longitudinal direction. Each of the first end portions 15 is provided with a plurality of waist-surrounding elastic members 17 extending in the waist-circumferential direction and secured thereto in a stretched state. Each of the second end portions 16 is folded back toward the inner surface of the associated first end portion 15 so that these first and second end portions 15, 16 may be overlaid together. The first and second end portions 15, 16 are defined by portions of the inner and outer sheets 13, 14 constituting the base sheet assembly 2.

A distal end zone 18 of the second end portion 16 extending beyond the waist-surrounding elastic members 17 in the waist-circumferential direction is secured to the associated first end portion 15. The first and second end portions 15, 16 respectively have transversely opposite lateral zones 19, 20 lying outside the side edges 8 of the panel 3 as viewed in the waist-circumferential direction and intermediate zones 21, 22 bordered by the lateral zones 19, 20 and the distal end zone 18. The first and second end portions 15, 16 are joined in the respective lateral zones 19, 20 thereof. On the other hand, these first and second end portions 15, 16 are not joined in the respective intermediate zones 21, 22 thereof, i.e., these end portions 15, 16 are let free from each other.

In the distal end zone 18 of the second end portion 16, the inner sheet 13 folded back onto itself has its inner surface overlaid and joined together by means of a hot melt adhesive 28. In the lateral zones 19, 20 of the first and second end portions 15, 16 also, the inner sheet 13 folded back onto itself has its inner surface overlaid and joined together by means of a hot melt adhesive 29. The waist-surrounding elastic members 17 are interposed between the inner and outer sheets 13, 14 and secured to opposite surfaces of these sheets 13, 14 by means of a hot melt adhesive 30.

In the crotch region 5 of the base sheet assembly 2, a peripheral edge portion 12a of the leg-hole 12 is provided with a plurality of leg-surrounding elastic members 23 extending in the leg-circumferential direction in a stretched state. The leg-surrounding elastic members 23 are interposed between the inner and outer sheets 13, 14 and bonded to at least one of these sheets 13, 14 by means of a hot melt adhesive (not shown).

The panel 3 is shaped in a rectangle which is relatively long in the longitudinal direction and comprises a liquid-pervious topsheet 24 facing the wearer's body, a liquid-impervious backsheet 25 facing away from the wearer's body and a liquid-absorbent core 26 interposed between these top- and backsheets 24, 25. On the panel 3, the backsheet 25 has its entire outer surface intermittently bonded to the inner surface of the inner sheet 13 by means of hot melt adhesive (not shown). Alternatively, the outer surface of the backsheet 25 may be intermittently bonded to the inner surface of the inner sheet 13 only along the longitudinally opposite ends 7 of the panel 3 and the other zones of the panel 3 may be let free from the inner sheet 13. The top- and backsheets 24, 25 are overlaid and joined along peripheral edges thereof.

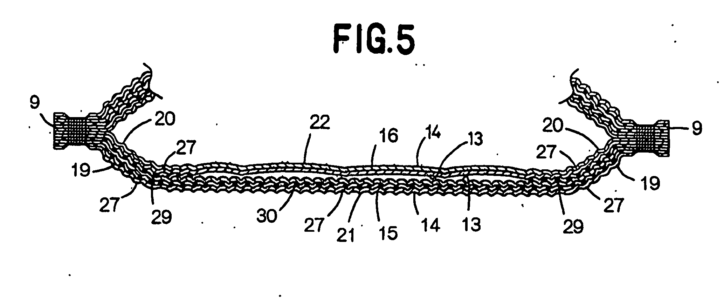

Fig. 5 is a sectional view taken along a line C-C in Fig. 1. As will be apparent from Fig. 5, contraction of the waist-surrounding elastic members 17 of the article 1A in the waist-circumferential direction causes the transversely opposite lateral zones 19, 20 of the first and second end portions 15, 16 as well as the intermediate portion 21 of the first end portion 15 to be formed with a plurality of gathers 27 (fine wrinkles). In the case of the article 1A, the intermediate zone 21 of the first end portion 15 and the intermediate zone 22 of the second end portion 16 are let free from each other, so the intermediate zone 22 of the second end portion 16 will slightly undulate even when the waist-surrounding elastic members 17 contract.

The intermediate zone 22 of the second end portion 16 which is slightly undulating as has been described above comes in direct contact with the wearer's skin as the article 1A is put on the wearer's body. With an advantageous consequence, the intermediate zone 22 of the second end portion 16 gives the wearer a comfortable touch, on one hand, and there is no anxiety that the gathers 27 formed in the intermediate zone 21 of the first end portion 15 might come in direct contact with the wearer' s skin and thereby deteriorate a touch of the article 1A. Furthermore, there is no possibility that the intermediate zone 21 of the second end portion 16 might leave any mark of compression on the wearer's skin due to gathers. The undulation appearing in the intermediate zone 22 of the second end portion 16 is not remarkable and therefore this intermediate zone 22 can be held in close contact with the wearer's skin without leaving a gap between the intermediate zone 22 and the wearer's body. In this way, it is not likely that an excessive amount of bodily discharges which can not be absorbed by the panel 3 might leak out from the article 1A beyond the intermediate zone 22.

In the article 1A, the transversely opposite lateral zones 19 of the second end portion 16 are formed with a plurality of the gathers 27 and therefore a gap is left between these lateral zones 19 of the second end portion 16 and the wearer's body. Such gap allows humidity generated within the article 1A to be evacuated from the article 1A and thereby allows undesirable stuffiness possibly generated within the article 1A to be reliably avoided.

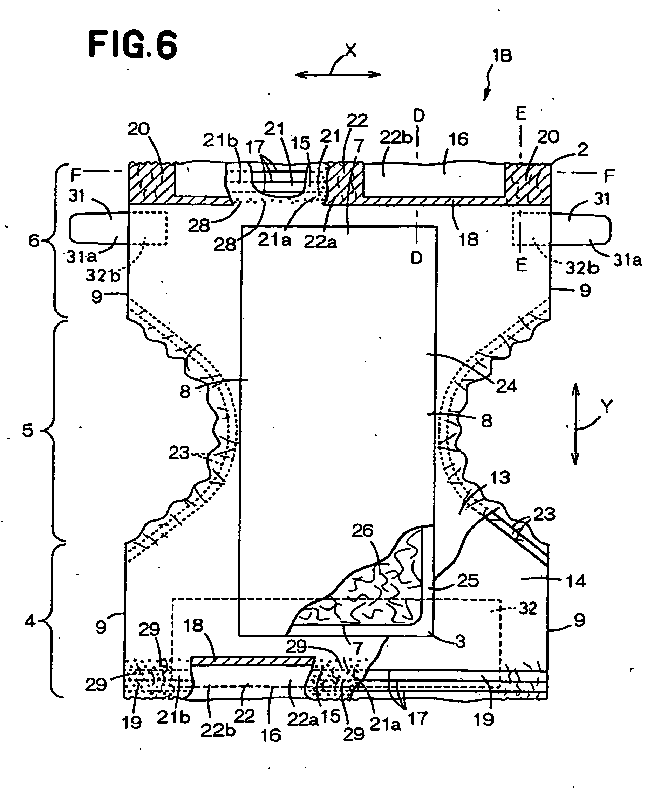

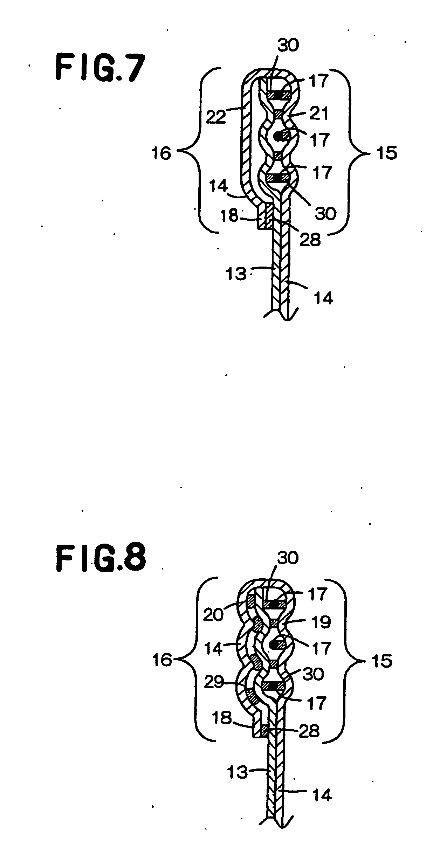

Fig. 6 is a perspective view showing a wearing article 1B according to another embodiment of the invention, Fig. 7 is a sectional view taken along a line D-D in Fig. 6 and Fig. 8 is a sectional view taken along a line E-E in Fig. 6. In Fig. 6, a waist-circumferential direction is indicated by an arrow X and a longitudinal direction is indicated by an arrow Y.

The article 1B comprises a base sheet assembly 2 and a liquid-absorbent panel 3 attached to an inner side of the base sheet assembly 2. The base sheet assembly 2 defines front and rear waist regions 4, 6 opposed to each other and a crotch region 5 extending between these waist regions 4, 6. The panel 3 extends between the front and rear waist regions 4, 6 of the base sheet assembly 2.

The base sheet assembly 2 comprises the inner sheet 13 (first sheet) facing the panel 3 and the outer sheet 14 (second sheet) facing away from the panel 3, i.e., lying outside the inner sheet 13. The inner sheet 13 and the outer sheet 14 are made of hydrophobic fibrous nonwoven fabric. The inner sheet 13 and the outer sheet 14 are joined by means of hot melt adhesive (not shown) intermittently applied on at least one of these sheets 13, 14.

The base sheet assembly 2 has the first end portions 15 extending outward beyond longitudinally opposite ends 7 of the panel 3 in the longitudinal direction and second end portions 16 extending further outward beyond the respective first end portions 15 in the longitudinal direction. Each of the first end portions 15 is provided with a plurality of waist-surrounding elastic members 17 extending in the waist-circumferential direction and secured thereto in a stretched state. Each of the second end portions 16 is folded back toward the inner surface of the associated first end portion 15 so that these first and second end portions 15, 16 may be overlaid each other. Each of the first end portions 15 is formed by the inner and outer sheets 13, 14 and each of the second end portions 16 is formed by the outer sheet 14 alone.

A distal end zone 18 of the second end portion 16 extending beyond the waist-surrounding elastic members 17 in the waist-circumferential direction is secured to the associated first end portion 15. The first and second end portions 15, 16 respectively have transversely opposite lateral zones 19, 20 lying outside the side edges 8 of the panel 3 as viewed in the waist-circumferential direction and intermediate zones 21, 22 bordered by the lateral zones 19, 20 and the distal end zone 18. The first and second end portions 15, 16 are joined in the respective lateral zones 19, 20 thereof. On the other hand, in the intermediate zones 21, 22, these first and second end portions 15, 16 are joined only in respective middle zones 21a, 22a of the intermediate zones 21, 22 thereof, i.e., these end portions 15, 16 are let free from each other in the other zones 21b, 22b of the end portions except for these middle zones 21a, 22a.

In the distal end zone 18 of the second end portion 16, the outer surface of the inner sheet 13 and the inner surface of the outer sheet 14 are overlaid and joined by means of a hot melt adhesive 28. In the lateral zones 19, 20 and the middle regions 21a, 22a in the intermediate zones 21, 22 of these first and second end portions 15, 16 also, the outer surface of the inner sheet 13 and the inner surface of the outer sheet 14 are joined together by means of a hot melt adhesive 29. The waist-surrounding elastic members 17 are interposed between the inner and outer sheets 13, 14 and secured to these sheets 13, 14 by means of a hot melt adhesive 30.

A peripheral edge of the base sheet assembly 2 in the crotch region 5 is provided with a plurality of leg-surrounding elastic members 23 extending in the leg-circumferential direction in a stretched state. The leg-surrounding elastic members 23 are interposed between the inner and outer sheets 13, 14 and bonded to at least one of opposite surfaces of these sheets 13, 14 by means of a hot melt adhesive (not shown).

The panel 3 comprises a liquid-pervious topsheet 24, a liquid-impervious backsheet 25 and a liquid-absorbent core 26 interposed between these top- and backsheets 24, 25. The backsheet 25 has its entire outer surface intermittently joined to an inner surface of the inner sheet 13. The top-and backsheets 24, 25 are overlaid and joined along peripheral edges thereof.

In the rear waist region 6, the base sheet assembly 2 is provided with a pair of tape fasteners 31, respectively, extending outward from the respective lateral marginal edges 9 in the waist-circumferential direction. Each of these tape fasteners 31 has a free end portion 31a coated with a self-adhesive (not shown) and a fixed end portion 31b fixed to the rear waist region 6. In the front waist region 4, the outer sheet 14 is provided on its outer surface with a rectangular tape strip 29 attached thereto so that the free end portions 31a of the respective tape fasteners 31 may be releasably anchored on this target tape strip 32. Both the tape fasteners 31 and the target tape strip 32 are formed by flexible plastic film.

To put the article 1B on the wearer' s body, the rear waist region 6 is placed upon the front waist region 4 so that the lateral marginal edges 9 of the rear waist region 6 may be substantially aligned with the lateral marginal edges 9 of the front waist region 4 and then the free end portions 31a of the respective tape fasteners 31 are anchored on the target tape strip 32 by means of a self-adhesive to connect the front and rear waist regions 4, 6 to each other. Upon connection of the front and rear waist regions 4, 6 to each other, the article 1B is formed with the waist-hole and a pair of the leg-holes (not shown).

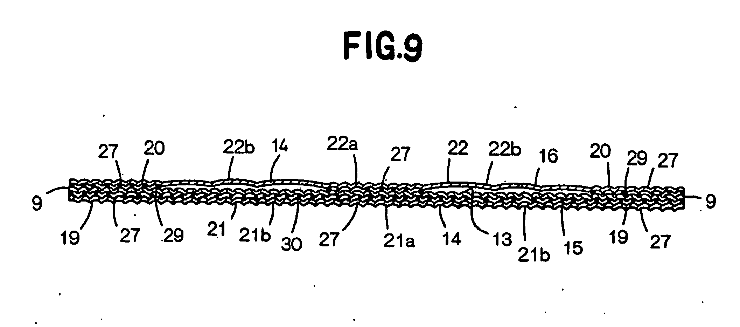

Fig. 9 is a sectional view taken along a line F-F in Fig. 6. As will be apparent from Fig. 9, contraction of the waist-surrounding elastic members 17 of the article 1B in the waist-circumferential direction causes the transversely opposite lateral zones 19, 20 of the first and second end portions 15, 16 as well as the middle zones 21a, 22a of the intermediate portion 21, 22 of the first and second end portion 15, 16 to be formed with a plurality of gathers 27 (fine wrinkles). In the case of the article 1B, these end portions 15, 16 are joined only in the middle zones 21a, 22a of the intermediate zone 21, 22 of the first and second end portions 15, 16, i.e., these end portions 15, 16 are let free from each other in the other zones 21b, 22b of the intermediate zones 21, 22 except for the middle zones 21a, 22a, so the undulation appearing along the other zone 22b of the intermediate zone 22 of the second end portion 16 will not be remarkable even when the waist-surrounding elastic members 17 contract.

In the case of the article 1B, the undulation appearing in the other zone 22b of the second end portion 16 is not remarkable as has been described above and the other zone 22b of the second end portion 16 coming in direct contact with the wearer's skin as the article 1B is put on the wearer's body ensures a comfortable touch and there is no possibility that the other zone 22b of the second end portion 16 might leave any mark of compression on the wearer's skin due to gathers. Furthermore, the other zone 22b of the second end portion 16 can be held in close contact with the wearer's skin without leaving a gap between the other zone 22b and the wearer' s body. In this way, it is not likely that an excessive amount of bodily discharges which can not be absorbed by the panel 3 might leak out from the article 1B beyond the other zone 22b.

In the article 1B, the second end portion 16 is formed by the outer sheet 14 and, compared to the case in which the second end portion 16 is formed by the inner and outer sheets 13, 14, stiffness of the second end portion 16 is sufficiently reduced not only to improve the touch of the second end portion 16 but also to ensure that the other zone 22b of the second end portion 16 can be reliably held in close contact with the wearer's skin.

In the case of the article 1B, the lateral zones 20 and the middle zone 22a of the intermediate zone 22 of the second end portion 16 are secured to the first end portion 15. This arrangement ensures that the first and second end portions 15, 16 are not separated from each other even when the waist-surrounding elastic members 17 are stretched in order to put the article 1B on the wearer's body. If the first and second end portions 15, 16 are separated from each other, the article would be put on the wearer' s body with the end portions 15, 16 lapped in two- or three-fold. Since the first and second end portions 15, 16 are not separated from each other in the article 1B, it is not likely that the first and second end portions 15, 16 might be lapped in two- or three-fold as the article 1B is put on the wearer's body.

A stock material for the inner and outer sheets 13, 14 may be selected from the group consisting of a breathable but liquid-impervious plastic film, a composite nonwoven fabric comprising two or more layers of hydrophobic fibrous nonwoven fabric placed upon one another and a composite sheet comprising a hydrophobic fibrous nonwoven fabric and a breathable but liquid-impervious plastic film placed upon each other.

A stock material for the topsheet 24 may be selected from the group consisting of a hydrophilic fibrous nonwoven fabric, a hydrophobic fibrous nonwoven fabric having a plurality of perforations and a plastic film having a plurality of fine perforations. A stock material for the backsheet 25 may be selected from the group consisting of a hydrophobic fibrous nonwoven fabric, a breathable but liquid-impervious plastic film, a composite nonwoven fabric comprising two or more fabric layers of hydrophobic fibrous nonwoven and a composite sheet comprising a hydrophobic fibrous nonwoven fabric and a breathable but liquid-impervious plastic film placed upon each other.

The nonwoven fabric may be selected from the group consisting of those obtained by spun lace-, needle punch-, melt blown-, thermal bond-, spun bond-, chemical bond- and air-through-processes. Component fibers of the nonwoven fabric may be selected from the group consisting of polyolefine-, polyester- and polyamide-based fibers and core-and-sheath type or side-by-side type conjugated fibers of polyethylene/polypropylene and polyethylene/polyester.

The core 26 comprises a mixture of fluff pulp and super-absorbent polymer particles or a mixture of fluff pulp, super-absorbent polymer particles and thermoplastic synthetic resin fibers each compressed to a given thickness. The core 26 is preferably entirely covered with a liquid-pervious sheet such as a tissue paper or a hydrophilic fibrous nonwoven fabric in order to prevent the core 26 from getting out of shape and to prevent the polymer particles from falling off therefrom. The polymer particles may be selected from the group consisting of starch-based polymer particles, cellulose-based polymer particles and synthetic polymer particles.

Joining of the top- and backsheets 24, 25 and attaching of the core 26 to the top- and backsheets 24, 25 may be carried out using a hot melt adhesive or welding technique such as heat-sealing or sonic sealing technique.

In these articles 1A, 1B illustrated as the particular embodiments, it is possible to form the second end portion 16 of the base sheet assembly 2 by the inner sheet 13 and it is also possible to secure the waist-surrounding elastic members 17 to the inner surface of the inner sheet 13 in a stretched state.

In the disposable wearing article according to the present invention, the intermediate zones of the first and second end portions are let free from each other, so the intermediate zone of the second end portion is not formed with gathers and ensures a comfortable touch even when the waist-surrounding elastic members contract. Furthermore, there is no possibility that the intermediate zone of the second end portion might leave any mark of compression on the wearer's skin due to gathers. Since this intermediate zone can be held in close contact with the wearer's skin without leaving a gap between the intermediate zone and the wearer's body, it is not likely that an excessive amount of bodily discharges which can not be absorbed by the panel might leak out from the article beyond the intermediate zone.

In this article, the transversely opposite lateral zones of the second end portion are formed with a plurality of the gathers and therefore a gap is left between these lateral zones of the second end portion and the wearer's body. Such gap allows humidity generated within the article to be evacuated from the article and thereby allows an undesirable stuffiness possibly generated within the article to be reliably avoided.

In the case of the article arranged so that the intermediate zones of the first and second end portions are locally secured, it is not likely that the first and second end portions might be separated from each other and be lapped in two- or three-fold as the article is put on the wearer's body.

## Claims

1. A disposable wearing article generally comprising a base sheet assembly (2) defining front and rear walat regions (4, 6) and
a crotch region (5) extending between said waist regions (4, 6) and
a liquid-absorbent panel (3) attached to an inner side of said base sheet assembly (2) so as to extend over said crotch region (5) further Into said front (4) and rear waist (5) rogions,
sold base sheet assembly (2) having
first end portions (15) extending outward beyond longitudinally opposite ends (7) of said panel (3) lying in said front and rear waist regions in a longitudinal direction and
second end portions (16) extending further outward beyond said first end portions (15) in said longitudinal direction,
each of said first end portions (15) being provided with a waist-surrounding elastic member (17) extending in a waist-circumferential direction in a stretched state,
each of said second end portions (16) being folded back onto an inner surface of associated one of said first end portions (15) and
distal end zones (18) of said second end portions (16) extending in said waist-circumferential direction being secured to said first end portions (15) so that
said second end portions (16) cover said waist-surrounding member (17),
said disposable wearing article further comprising:
said first and second end portions (15, 16) respective having lateral zones (19, 20) lying outside transversely opposite side edges (8) of said panel as viewed in said waist-circumferential direction and
intermediate zones (21, 22) bordered by said distal end zones (18) and said lateral zones; and
said first and second end portions (15, 16) being joined together in said lateral zones (19, 20) and let free from each other or are partially joined in respective said intermediate zones (21, 22).

2. The wearing article according to Claim 1, wherein said base sheet assembly comprises a first sheet facing said panel and a second sheet fac-ing away from said panel and said second portions are formed by at least one of said first and second sheets.

3. The wearing article according to Claim 2, wherein said waist-surrounding elastic member is interposed between said first and second sheets.

4. The wearing article according to any one of Claims 1 through 3, wherein said first and second sheets constituting said base sheet assembly are joined together in the vicinity of peripheral edges thereof lying outside said transversely opposite lateral side edges of said panel and said wearing article is formed with a waist-hole and a pair of leg-holes.

5. The wearing article according to any one of Claims 1 through 4, wherein said panel comprises a liquid-pervious top sheet facing the wearer's body, a liquid-impervious back sheet facing away from the wearer's body and a liquid-absorbent core interposed between said top- and back sheets.

## Patentansprüche

1. Wegwerftrageartikel im Wesentlichen umfassend eine Basislagenanordnung (2), die vordere und hintere Taillenbereiche (4, 6) bestimmt, und
einen Schrittbereich (5), der sich zwischen den Taillenbereichen (4, 6) erstreckt und
ein flüssigkeitsabsorbierendes Panel (3), das an einer inneren Seite der Basislagenanordnung (2) befestigt ist, um sich über den Schrittbereich (5) weiter in die vorderen und hinteren Taillenbereiche (4, 6) zu erstrecken,
wobei die Basislagenanordnung (2)
erste Endabschnitte (15), die sich auswärts über longitudinale gegenüber liegende Enden (7) des Panels (3) erstrecken, die in den vorderen und hinteren Taillenbereichen in longitudinaler Richtung liegen, und
zweite Endabschnitte (16), die sich in longitudinaler Richtung weiter auswärts über die ersten Endabschnitte (15) erstrecken,
wobei jeder der ersten Endabschnitte (15) ein die Taille umrundendes elastisches Element (17) aufweist, das sich in einem gedehnten Zustand in einer die Taille umrundenden Richtung erstreckt,
wobei jeder der zweiten Endabschnitte (16) auf eine innere Fläche des jeweils zugehörigen ersten Endabschnitts (15) zurückgefaltet ist, und
distale, sich in einer die Taille umrundenden Richtung erstreckende Endbereiche (18) der zweiten Endabschnitte (16) sind an den ersten Endabschnitten (15) befestigt, so dass die zweiten Endabschnitte (16) das die Taille umrundende Element (17) bedecken, aufweist, wobei
der Wegwerftrageartikel ferner umfasst:
die ersten und zweiten Endabschnitte (15, 16) weisen jeweils laterale Bereiche (19, 20) auf, die, in taillenumrundender Richtung gesehen, außerhalb transversal gegenüber liegender Seitenkanten (8) des Panels liegen, und
die ersten und zweiten Endabschnitte (15, 16) sind in den lateralen Bereichen (19, 20) miteinander verbunden und in den jeweiligen Zwischenbereichen (21, 22) voneinander gelöst oder teilweise verbunden.

2. Wegwerftrageartikel nach Anspruch 1, wobei die Basislagenanordnung eine erste, dem Panel zugewandte Lage und eine zweite, dem Panel abgewandte Lage umfasst, und die zweiten Abschnitte durch zumindest eine der ersten und zweiten Lage gebildet ist.

3. Wegwerftrageartikel nach Anspruch 2, wobei das die Taille umrundende Element zwischen der ersten und zweiten Lage angeordnet ist.

4. Wegwerftrageartikel nach irgendeinem der Ansprüche 1 bis 3, wobei die ersten und zweiten Lagen, welche die Basislagenanordnung bilden, in der Nähe ihrer Außenkante, die außerhalb der transversalen gegenüber liegenden lateralen Seitenkanten des Panels liegen, verbunden sind, und wobei der Wegwerftrageartikel mit einer Taillenöffnung und einem Paar Beinöffnungen gebildet ist.

5. Wegwerftrageartikel nach irgendeinem der Ansprüche 1 bis 4, wobei das Panel eine flüssigkeitsdurchlässige Oberlage, die dem Körper des Trägers zugewandt ist, eine flüssigkeitsundurchlässige Unterlage, die dem Körper des Trägers abgewandt ist, und einen flüssigkeitsabsorbierenden Kern, der zwischen der Oberlage und der Unterlage angeordnet ist, umfasst.

## Revendications

1. Article absorbant jetable comprenant une assemblage de la feuille de base (2) qui détermine l'avant et à l'arrière des régions de ceinture (4,6) et une région d'entrejambe (5)lequel s'étendant entre les régions de ceinture (4,6)et
un panneau absorbant les liquides (3), qui est fixé à un côté intérieur de ladite l'assemblage de la feuille de base(2)pour s'étendre à travers la région d'entrejambe (5) en outre dans la région de ceinture avant et arrière (4,6)
dans lequel l'assemblage de la feuille de base (2)comprends premières parties d'extrémité (15) s'étendant vers l'extérieur sur longitudinal opposé extrémités (7) du panneau (3) qui se trouvent à l'avant et à l'arrière des régions de ceinture dans la direction longitudinale, et
deuxièmes parties d'extrémité (16) s'étendant dans une direction longitudinale plus loin au-delà des premières parties d'extrémité (15),
dans lequel chacune desdites premières parties d'extrémité (15) comprend un élément élastique de ceinture (17), qui est dans un état expansé, s'étendant dans une direction de ceinture en orbite,
dans lequel chacune desdites secondes parties d'extrémité (16) est replié sur une surface intérieure de la première partie d'extrémité (15) correspondant, et des parties d'extrémité(18)distales des secondes parties d'extrémité (16) s'étendant dans une direction de ceinture en orbite sont fixés aux premières parties d'extrémité (15) de sorte que les secondes parties d'extrémité (16) recouvrent l'élément élastique de ceinture (17)en orbite, dans lequel
l'article absorbant jetable comprenant en plus:
les parties d'extrémité premier et deuxième (15,16) ont chacune des régions latérales (19, 20), qui sont situées en dehors des bords latéraux du panneau transversalement opposés (8) vue dans une direction orbitale du taille , et
les zones intermédiaires (21, 22) délimitée par les zones d'extrémité distale (18) et les régions latérales, et
les parties d'extrémité premier et deuxième (15, 16) sont reliés l'un à l'autre dans les régions latérales (19,20) et sont séparés les uns des autres ou partiellement reliées dans les zones intermédiaires (21, 22) respectives.

2. Article absorbant jetable selon la revendication 1, dans lequel l'assemblage de feuille de base comprenant une première couche faisant face au panneau et une deuxième couche en face opposée du panneau et les deuxièmes sections sont formées par au moins une desdites première et deuxième couches.

3. Article absorbant jetable selon la revendication 2, dans lequel l'élément de ceinture en orbite est disposé entre la première et la deuxième couche.

4. Article absorbant jetable selon l'une quelconque des revendications 1 à 3, dans lequel les premières et deuxièmes couches, qui forment l'assemblage de la feuille de base et sont reliés près de ses bords extérieurs, qui sont situées à l'extérieur des bords latéraux du panneau traverses opposées et dans lequel l'article absorbant jetable est formée avec une ouverture pour la taille et une paire d'ouvertures de jambe.

5. Article absorbant jetable selon l'une quelconque des revendications 1 à 4, dans lequel le panneau comprenant une feuille perméable aux liquides supérieure qui est tournée vers le corps du porteur et une feuille imperméable aux liquides inférieure qui est face à l'écart du corps du porteur et un coeur absorbant le liquide qui est disposé entre la feuille supérieure et la feuille inférieure.
